(19) Europäisches Patentamt
European Patent Office
Office européen des brevets

(11) Publication number: 0 098 161
A2

# (12) EUROPEAN PATENT APPLICATION

(21) Application number: 83303758.3

(22) Date of filing: 29.06.83

(51) Int. Cl.[3]: C 07 D 501/24
A 61 K 31/545

(30) Priority: 30.06.82 GB 8218874
02.08.82 GB 8222236

(43) Date of publication of application:
11.01.84 Bulletin 84/2

(84) Designated Contracting States:
AT BE CH DE FR GB IT LI LU NL SE

(71) Applicant: GLAXO GROUP LIMITED
Clarges House 6-12 Clarges Street
London W1Y 8DH(GB)

(72) Inventor: Looker, Brian Edgar
28 Middleton Avenue
Greenford Middlesex(GB)

(74) Representative: Holmes, Michael John et al,
Frank B. Dehn & Co. European Patent Attorneys Imperial House 15-19 Kingsway
London, WC2B 6UZ,(GB)

## (54) Cephalosporin compounds.

(57) Cephalosporin compounds of general formula

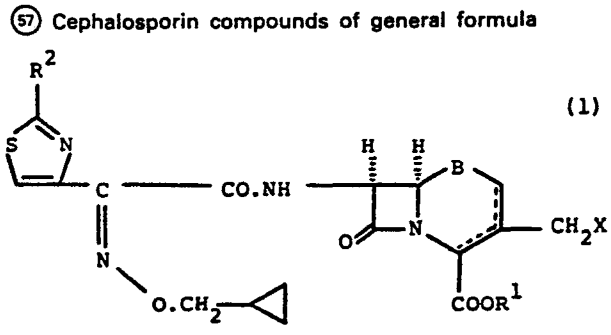

(1)

(I)

(wherein $R^1$ represents a hydrogen atom or a carboxyl blocking group, $R^2$ represents an amino or protected amino group, X represents a halogen atom or a hydroxyl or acetoxy group, B is S or S → O, and the dotted line bridging the 2-, 3- and 4-positions indicates that the compound is a ceph-2-em or ceph-3-em compound) and salts thereof. The compounds may be useful in the preparation of other cephalosporin compounds possessing antibiotic activity. Futhermore, ceph-3-em compounds of formula (I) in which $R^2$ is an amino group, B is S and X is hydroxy or acetoxy, as well as the non-toxic salts and non-toxic metabolically labile esters thereof, themselves exhibit antibiotic activity.

EP 0 098 161 A2

CK.141-279

"Cephalosporin Compounds"

This invention relates to improvements in or relating to cephalosporins. More particularly it relates to new cephalosporin compounds and derivatives thereof having valuable antibiotic activity and which may be used in the preparation of other cephalosporin antibiotic compounds.

The cephalosporin compounds in this specification are named with reference to "cepham" after J.Amer.Chem. Soc., 1962, 84, 3400, the term "cephem" referring to the basic cepham structure with one double bond.

Cephalosporin antibiotics are widely used in the treatment of diseases caused by pathogenic bacteria in human beings and animals, and are especially useful in the treatment of diseases caused by bacteria which are resistant to other antibiotics such as penicillin compounds, and in the treatment of penicillin-sensitive patients. In many instances it is desirable to employ a cephalosporin antibiotic which exhibits activity against both Gram-positive and Gram-negative microorganisms, and a significant amount of research has been directed to the development of various types of broad spectrum cephalosporin antibiotics.

Thus, for example in our British Patent Specification No. 1,399,086, we describe a novel class of cephalosporin antibiotics containing a 7β-(α-etherified oximino)-acylamido group, the oximino group having the syn configuration. This class of antibiotic compounds is characterised by high antibacterial activity against a range of Gram-positive and Gram-negative organisms coupled with particularly high stability to β-lactamases produced by various Gram-negative organisms.

The discovery of this class of compounds has stimulated further research in the same area in attempts

to find compounds which have improved properties, for example against particular classes of organisms especially Gram-negative organisms.

In British Patent Specification No. 1,604,971 a wide variety of cephalosporin antibiotics are disclosed in which the 7β-position side-chain may be selected from, inter alia, a 2-(2-aminothiazol-4-yl)-2-(etherified oxyimino)acetamido group, in which the etherifying group, amongst very many possible meanings, may be an alkyl group substituted by a cycloalkyl group, although there is no specific exemplification of compounds having such a group. The 3-position group may also be selected from a large number of alternatives including halogen atoms and hydroxymethyl and acetoxymethyl groups.

Other cephalosporin compounds possessing a 2-(2-aminothiazol-4-yl)-2-(etherified oxyimino)acetamido group in the 7β-position are disclosed in for example British Patent Specifications Nos. 1,576,625, 1,580,621, 2,029,824A and 2,027,692A.

We have discovered that by the selection of a syn 2-(2-aminothiazol-4-yl)-2-cyclopropylmethoxyimino-acetamido group at the 7β-position in combination with either a pyridiniummethyl group (wherein the pyridinium moiety is optionally substituted by a 3- or 4-carbamoyl group) or a carbamoyloxymethyl group (wherein the carbamoyl moiety is optionally N-substituted by a methyl or 2-chloroethyl group) at the 3-position, cephalosporin compounds having particularly advantageous activity against a wide range of commonly encountered pathogenic organisms may be obtained. In particular, these compounds have high activity against Gram-positive and Gram-negative organisms, including many β-lactamase producing strains, both in vitro and in vivo. The compounds also possess high stability to β-lactamases

produced by a range of Gram-positive and Gram-negative organisms. The compounds have been found to exhibit high activity against strains of Staphylococcus aureus Staphylococcus epidermidis and Streptococcus species coupled with high activity against various members of the Enterobacteriaceae, strains of Haemophilus influenzae and Acinetobacter calcoaceticus, as well as good activity against Pseudomonas species.

We have further found that the corresponding 3-hydroxymethyl, 3-acetoxymethyl and 3-halomethyl compounds and derivatives thereof are particularly convenient starting materials for the preparation of cephalosporin antibiotics having a syn 2-(2-aminothiazol-4-yl)-2-cyclopropylmethoxyiminoacetamido 7-side chain, including the above-mentioned compounds. We have also found that certain of the 3-hydroxymethyl and 3-acetoxymethyl compounds themselves possess interesting antibiotic properties.

Accordingly, we provide cephalosporin compounds of the general formula (I)

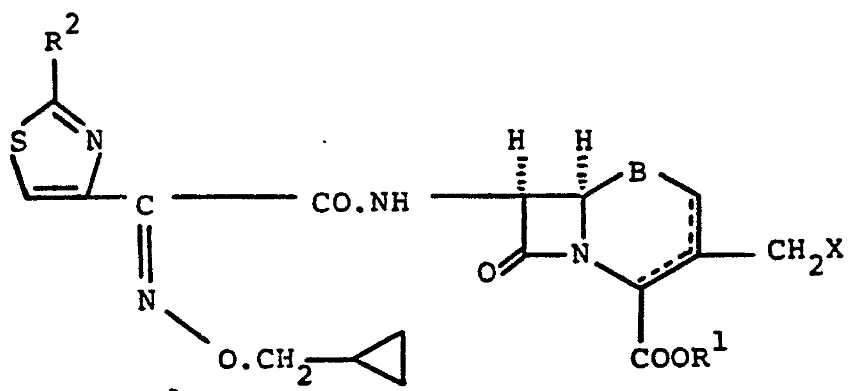

(I)

[wherein $R^1$ represents a hydrogen atom or a carboxyl blocking group e.g. the residue of an ester-forming aliphatic or araliphatic alcohol or an ester-forming phenol, silanol or stannanol (the said alcohol, phenol, silanol or stannanol preferably containing 1 to 20 carbon atoms); $R^2$ represents an amino or protected amino group; X represents a hydroxyl or acetoxy group or a halogen atom (e.g. a chlorine, bromine or iodine atom); B is >S or >S→O (α- or β-); and the dotted line bridging the 2-, 3- and 4-positions indicates that the compound is a ceph-2-em or ceph-3-em compound] and salts thereof.

The compounds according to the invention are *syn* isomers. The *syn* isomeric form is defined by the configuration of the 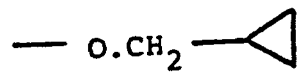 group with respect to the carboxamido group. In this Specification, the *syn* configuration is denoted structurally as

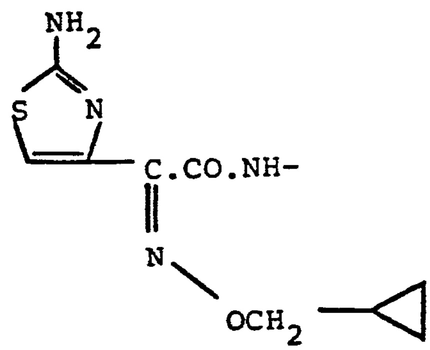

It will be understood that since the compounds according to the invention are geometric isomers, some admixture with the corresponding *anti* isomer may occur.

The invention also includes within its scope the solvates (especially the hydrates) of the compounds of formula (I) and of their salts.

The compounds according to the present invention may exist in tautomeric forms (for example in respect of the 2-aminothiazolyl group) and it will be understood that such tautomeric forms, e.g. the 2-iminothiazolinyl form, are included within the scope of the invention.

Ceph-3-em compounds according to the invention in which $R^2$ is an amino group, B is $>$S and X is a hydroxy or acetoxy group generally exhibit antibiotic activity. These and other compounds according to the invention may also be useful as intermediates or starting materials in the preparation of active compounds of the invention or other cephalosporin antibiotic compounds. It will be appreciated that salts of the compounds for use in medicine should be non-toxic. Similarly where $R^3$ is a carboxyl blocking group in compounds to be used in medicine, this should represent a metabolically labile non-toxic ester function.

Thus,active compounds according to the invention may be represented by the general formula

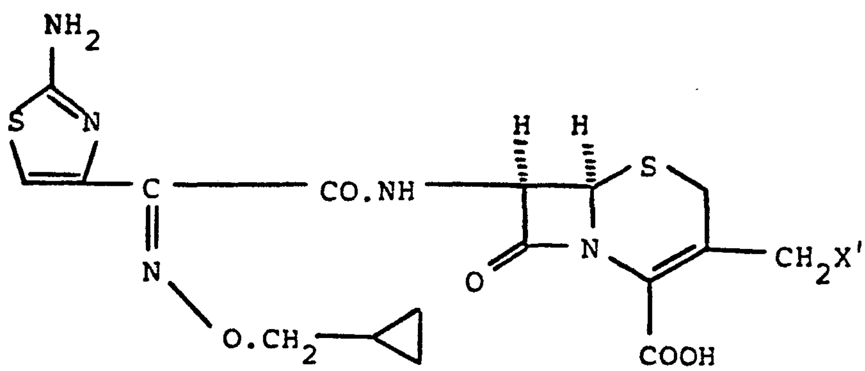

(Ia)

(wherein X' is a hydroxy or acetoxy group) and the non-toxic salts and non-toxic metabolically labile esters thereof.

The compounds of formula (Ia) and salts and esters thereof exhibit broad spectrum antibiotic activity both *in vitro* and *in vivo*. They have activity against both Gram-positive and Gram-negative organisms, including many β-lactamase producing strains. The compounds also possess stability to β-lactamases produced by a range of Gram-negative and Gram-positive organisms.

The active compounds have been found to exhibit good activity against strains of *Staphylococcus aureus* and *Staphylococcus epidermidis* including penicillinase producing strains of these Gram-positive bacteria. This is coupled with good activity against various members of the Enterobacteriaceae (e.g. strains of *Escherichia coli*, *Klebsiella pneumoniae*, *Citrobacter diversus*,

Enterobacter cloacae, Serratia marcescens, Proteus mirabilis and indole-positive Proteus organisms such as Proteus vulgaris, Proteus morganii and Providence species), strains of Haemophilus influenzae, and Acinetobacter calcoaceticus as well as activity against Pseudomonas species.

Non-toxic salt derivatives which may be formed by reaction of the carboxyl group present in the compounds of formula (Ia) include inorganic base salts such as alkali metal salts (e.g. sodium and potassium salts) and alkaline earth metal salts (e.g. calcium salts); amino acid salts (e.g. lysine and arginine salts); organic base salts (e.g. procaine, phenylethylbenzylamine, dibenzylethylenediamine, ethanolamine, diethanolamine and N-methylglucosamine salts). Other non-toxic salt derivatives include acid addition salts, e.g. formed with hydrochloric, hydrobromic, sulphuric, nitric, phosphoric, formic and trifluoroacetic acids. The salts may also be in the form of resinates formed with, for example, a polystyrene resin or cross-linked polystyrene divinylbenzene copolymer resin containing amino or quaternary amino groups or sulphonic acid groups, or with a resin containing carboxyl groups, e.g. a polyacrylic acid resin. Soluble base salts (e.g. alkali metal salts such as the sodium salt) of the compounds of formula (Ia) may be used in therapeutic applications because of the rapid distribution of such salts in the body upon administration. Where, however, insoluble salts of compounds (Ia) are desired in a particular application, e.g. for use in depot preparations, such salts may be formed in conventional manner, for example with appropriate organic amines.

Non-toxic metabolically labile ester derivatives which may be formed by esterification of the carboxyl group in the compound of formula (Ia) include acyloxyalkyl esters, e.g. lower alkanoyloxy-methyl or -ethyl esters such as acetoxy-methyl or -ethyl or pivaloyloxymethyl esters. In addition to the above ester derivatives,

the present invention includes within its scope the compounds of formula (Ia) in the form of other physiologically acceptable equivalents, i.e. physiologically acceptable compounds which, like the metabolically labile esters, are converted in vivo into the parent antibiotic compound of formula (Ia).

Suitable salts and esters of formula (I) include the non-toxic salts and esters mentioned above as well as other salts and esters such as the salts with toluene-p-sulphonic and methanesulphonic acids or the esters with t-butyl or diphenylmethyl esterifying groups. Such salts and esters may be employed as intermediates in the preparation and/or purification of the compounds of formula (Ia) and of other cephalosporin antibiotic compounds, for example in the processes described below.

The active compounds of the invention may be used for treating a variety of diseases caused by pathogenic bacteria in human beings and animals, such as respiratory tract infections and urinary tract infections.

As indicated above the compounds according to the invention may be used as starting materials for the preparation of other cephalosporin antibiotic compounds. In particular, they may be used for preparing other cephalosporin antibiotics possessing a syn 2-(2-aminothiazol-4-yl)-2-cyclopropylmethoxyiminoacetamido group at the 7β-position and a different substituent, for example a 1-pyridiniummethyl or carbamoyloxymethyl group, at the 3-position. Examples of cephalosporin antibiotics which may be prepared in this way include (6R,7R)-7-[(Z)-2-(2-aminothiazol-4-yl)-2-cyclopropylmethoxyiminoacetamido]-3-(1-pyridiniummethyl)ceph-3-em-4-carboxylate (in which the pyridinium group may be optionally substituted by a 3- or 4-carbamoyl group) and (6R,7R)-3-carbamoyloxymethyl-7-[(Z)-2-(2-aminothiazol-4-yl)-2-cyclopropylmethoxy-iminoacetamido]ceph-3-em-4-carboxylic acid (in which the carbamoyl group is optionally N-substituted by a methyl or 2-chloroethyl

group), as well as the non-toxic salts and non-toxic metabolically labile esters of these compounds.

According to another embodiment of the invention we provide a process for the preparation of compounds of the invention which comprises

(A) acylating a compound of the formula

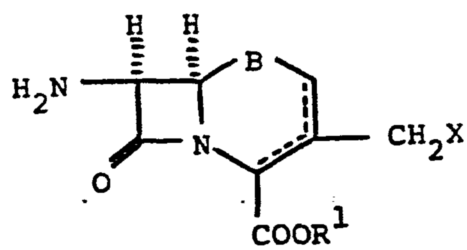

(II)

(wherein $R^1$, X, B and the dotted line are as hereinbefore defined) or a salt, e.g. an acid addition salt (formed with, for example, a mineral acid such as hydrochloric, hydrobromic, sulphuric, nitric or phosphoric acid or an organic acid such as methanesulphonic or toluene-p-sulphonic acid) or an N-silyl derivative thereof, with an acid of formula

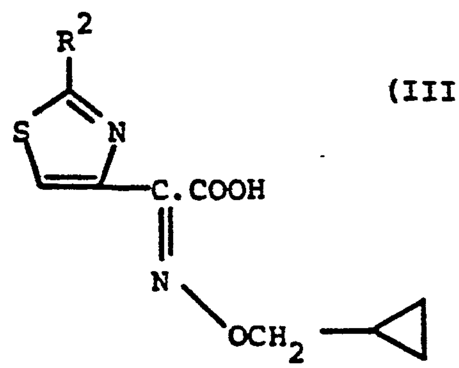

(III)

(wherein $R^2$ is as hereinbefore defined) or a salt thereof, or with an acylating agent corresponding thereto;

(B) (for the preparation of a compound in which X is acetoxy) reacting a compound of general formula (I) in which X is a hydroxy group or a salt thereof, with an acetylating agent serving to form an acetoxymethyl group at the 3-position;

or (C) (for the preparation of a compound in which X is a hydroxy group) 3-deacetylating a compound of general formula (I) in which X is an acetoxy group or a salt thereof; whereafter, if desired in each instance, any of the following reactions, in any appropriate sequence, are carried out:-

i) conversion of a $\Delta^2$-isomer into the corresponding $\Delta^3$-isomer,

ii) reduction of a compound wherein B is $>S \rightarrow O$ to form a compound wherein B is $>S$,

iii) conversion of a carboxyl group into an ester function e.g.a non-toxic metabolically labile ester function,

iv) formation of a salt function e.g. a non-toxic salt function, and

v) removal of any carboxyl blocking and/or N-protecting groups.

The above reactions i) to v) may be carried out in conventional manner. It will be appreciated that these reactions may, in particular, be employed when a compound of formula (Ia) is desired.

In the above-described process (A), the starting material of formula (II) is preferably a compound wherein B is $>S$ and the dotted line represents a ceph-3-em compound.

Acylating agents which may be employed in the preparation of compounds of formula (I) include acid halides, particularly acid chlorides or bromides. Such acylating agents may be prepared by reacting an acid (III) or a salt thereof with a halogenating

agent e.g. phosphorus pentachloride, thionyl chloride or oxalyl chloride.

Acylations employing acid halides may be effected in aqueous and non-aqueous reaction media, conveniently at temperatures of from -50 to +50°C, preferably -40 to +30°C, if desired in the presence of an acid binding agent. Suitable reaction media include aqueous ketones such as aqueous acetone, aqueous alcohols such as aqueous ethanol, esters such as ethyl acetate, halogenated hydrocarbons such as methylene chloride, amides such as dimethylacetamide, nitriles such as acetonitrile, or mixtures of two or more such solvents. Suitable acid binding agents include tertiary amines (e.g. triethylamine or dimethylaniline), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acids of formula (III) may themselves be used as acylating agents in the preparation of compounds of formula (I). Acylations employing acids (III) are desirably conducted in the presence of a condensing agent, for example a carbodiimide such as N,N'-dicyclohexylcarbodiimide or N-ethyl-N'-γ-dimethylaminopropylcarbodiimide; a carbonyl compound such as carbonyldiimidazole; or an isoxazolium salt such as N-ethyl-5-phenylisoxazolium perchlorate.

Acylation may also be effected with other amide-forming derivatives of acids of formula (III) such as, for example, an activated ester, a symmetrical anhydride or a mixed anhydride (e.g. formed with pivalic acid or with a haloformate, such as a lower alkylhaloformate). Mixed anhydrides may also be formed with phosphorus acids (for example phosphoric or phosphorous acids), sulphuric acid or aliphatic or aromatic sulphonic acids (for example toluene-p-sulphonic acid). An activated ester may conveniently be formed <u>in situ</u> using, for example, 1-hydroxybenzotriazole in the

presence of a condensing agent as set out above. Alternatively, the activated ester may be preformed.

Acylation reactions involving the free acids or their above-mentioned amide-forming derivatives are desirably effected in an anhydrous reaction medium, e.g. methylene chloride, tetrahydrofuran, dimethylformamide or acetonitrile.

An alternative method of activation is, for example, by reacting an acid of formula (III) with a solution or suspension preformed by adding a carbonyl halide, in particular oxalyl chloride or phosgene, or a phosphoryl halide such as phosphorus oxychloride to a solvent such as a halogenated hydrocarbon, for example methylene chloride, containing a lower acyl tertiary amide such as N,N-dimethylformamide. The activated form of the the acid of formula (III) may then be reacted with a 7-amino compound of formula (II) in a suitable solvent or mixture of solvents for example an alkanol such as an alcohol, e.g. aqueous ethanol or aqueous industrial methylated spirts. The acylation reaction may conveniently be effected at temperatures of from -50° to +50°C, preferably -40° to +30°C, if desired in the presence of an acid binding agent, for example as described above (e.g. triethylamine).

If desired, the above acylation reactions may be carried out in the presence of a catalyst such as 4-dimethylaminopyridine.

The acids of formula (III) and acylating agents corresponding thereto may, if desired, be prepared and employed in the form of their acid addition salts. Thus, for example, acid chlorides may conveniently be employed as their hydrochloride salts, and acid bromides as their hydrobromide salts.

Acetylation of 3-hydroxymethyl compounds according to process (B) may be effected by conventional methods, for example in an analogous manner to that described in British Patent Specification No. 1141293, i.e.

by blocking the 4-carboxy group (where this is not already blocked), acetylating the 3-hydroxymethyl group of the protected compound and, if desired, subsequently removing the blocking group.

Compounds according to the invention in which X represents a hydroxyl group can be prepared by deacetylation of the corresponding 3-acetoxymethyl compounds according to process (C), for example by hydrolysis of the 3-acetoxymethyl compounds, e.g. as described for example in British Patent Specifications Nos. 1,474,519 and 1,531,212. A particularly convenient method for the deacetylation of a 3-acetoxymethyl compound is by the enzymatic hydrolysis described in our British Patent Specification No. 1,531,212 e.g. using an esterase derived from Rhodosporidium toruloides.

The reaction product may be separated from the reaction mixture, which may contain, for example, unchanged cephalosporin starting material and other substances, by a variety of processes including recrystallisation, ionophoresis, column chromatography and use of ion-exchangers (for example by chromatography on ion-exchange resins) or macroreticular resins.

If desired, a $\Delta^2$-cephalosporin ester derivative obtained in accordance with the process of the invention may be converted into the corresponding $\Delta^3$-derivative by, for example, treatment of the $\Delta^2$-ester with a base, such as pyridine or triethylamine.

A ceph-2-em reaction product may also be oxidised to yield the corresponding ceph-3-em 1-oxide, for example by reaction with a peracid, e.g. peracetic or m-chloroperbenzoic acid; the resulting sulphoxide may subsequently be reduced as described hereinafter to yield the corresponding ceph-3-em sulphide.

Where a compound is obtained in which B is $>S \rightarrow O$ this may, if desired, be converted into the corresponding sulphide by, for example, reduction of the corresponding acyloxysulphonium or alkoxysulphonium salt prepared

*in situ* by reaction with e.g. acetyl chloride in the case of an acetoxysulphonium salt, reduction being effected by, for example, sodium dithionite or by iodide ion as in a solution of potassium iodide in a water-miscible solvent e.g. acetic acid, acetone, tetrahydrofuran, dioxan, dimethylformamide or dimethylacetamide. The reaction may be effected at a temperature of from -20° to +50°C.

An acid or a salt of formula (I) may, if desired, be converted to an ester derivative of formula (I), e.g. a metabolically labile ester derivative, by reaction with an appropriate esterifying agent such as an acyloxyalkyl halide (e.g. iodide) conveniently in an inert organic solvent such as dimethylformamide or acetone.

Base salts of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) in which $R^2$ is an amino group with the appropriate acid.

Where a compound of formula (I) is obtained as a mixture of isomers, the *syn* isomer may be obtained by, for example, conventional methods such as crystallisation or chromatography.

For use as starting materials for the preparation of compounds of general formula (I) according to the invention, compounds of general formula (III) and amide-forming derivatives e.g.acid halides and anhydrides corresponding thereto in their *syn* isomeric form or in the form of mixtures of the *syn* isomers and the corresponding *anti* isomers containing at least 90% of the *syn* isomer are preferably used.

Acids of formula (III) may be prepared by etherification of a compound of formula

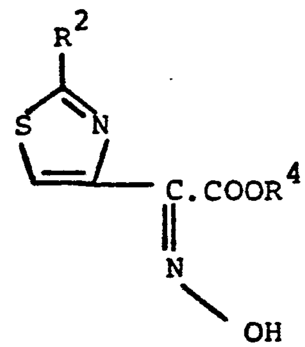

(VI)

(wherein $R^2$ is as hereinbefore defined and $R^4$ represents hydrogen or a carboxyl blocking group) or a salt thereof, by selective reaction with a compound of general formula

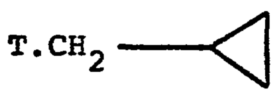

(VII)

(wherein T is halogen, such as chloro, bromo or iodo; sulphate; or sulphonate, such as tosylate), followed by removal of any carboxyl blocking group $R^4$. Separation of isomers may be effected either before or after such etherification. The etherification reaction is conveniently carried out in the presence of a base, e.g. potassium carbonate or sodium hydride, and is preferably conducted in an organic solvent, for example dimethylsulphoxide, a cyclic ether such as tetrahydrofuran or dioxan, or an N,N-disubstituted amide such as dimethylformamide. Under these conditions the configuration of the oxyimino group is substantially unchanged by the etherification reaction. When the compound of formula (VI) is employed in the form of a free acid or a salt with a base, the etherfication reaction is generally carried out in the presence of a strong base e.g. potassium t-butoxide, sufficient base being added to form a dianion. Furthermore, the reaction should be effected in the presence of a base if an acid addition salt of a compound of formula (VI) is used, the amount of the base being sufficient to neutralise rapidly the acid in question.

Acids of formula (III) may also be prepared by reaction of a compound of formula

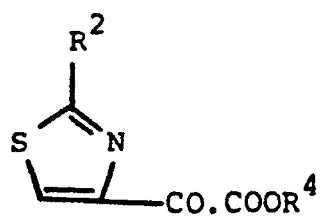

(VIII)

(wherein $R^2$ and $R^4$ are as hereinbefore defined) with a compound of formula

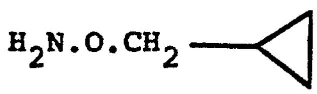

(IX)

followed by removal of any carboxyl blocking group $R^4$, and where necessary the separation of <u>syn</u> and <u>anti</u> isomers.

The acids of formula (III) may be converted into the corresponding acid halides and anhydrides and acid addition salts by conventional methods, for example as described hereinabove.

The starting materials of formula (II) in which X is a hydroxyl group may be prepared, for example, by deacetylation of the corresponding 3-acetoxymethyl compound as described in British Patent Specifications Nos. 1,474,519 and 1,531,212.

The starting materials of formula (II) in which X is a halogen atom may be prepared in conventional manner. For example, a 7β-protected amino-3-methylceph-3-em-4-carboxylic acid ester 1β-oxide may be halogenated, followed by removal of the 7β-amino protecting group and, if desired, reduction of the 1β-oxide function and/or removal of the ester group.

A further method for the preparation of the starting materials of formula (II) comprises deprotecting a corresponding protected 7β-amino compound in conventional manner, e.g. using $PCl_5$.

When $R^2$ in the above compounds represents a protected amino group, this may be formed by any conventional method,for example by tritylation, acylation (e.g. chloroacetylation or formylation) and protonation of the amino group. If desired, an amino protecting group may be removed in any convenient way which does not cause breakdown of the compound, e.g. in the case of a trityl group by using an optionally halogenated carboxylic acid, e.g. acetic acid, formic acid, chloroacetic acid or trifluoroacetic acid or using a mineral acid, e.g. hydrochloric acid or mixtures of such acids, preferably in the presence of a protic solvent such as water, or, in the case of a chloroacetyl group, by treatment with thiourea.

Carboxyl blocking groups for $R^1$ present in the compounds of formula (I) or used in the preparation of necessary starting materials are desirably groups which may readily be split off at any suitable stage in any reaction sequence, conveniently at the last stage in a preparation of a cephalosporin antibiotic. It may, however, be convenient in some instances to employ non-toxic metabolically labile carboxyl blocking groups such as acyloxy-methyl or -ethyl groups (e.g. acetoxy-methyl or-ethyl or pivaloyloxymethyl) and retain these in the final product to give an appropriate ester derivative of a cephalosporin antibiotic e.g. a compound of formula (Ia).

Suitable carboxyl blocking groups are well known in the art, a list of representative blocked carboxyl groups being included in British Patent No. 1,399,086. Preferred blocked carboxyl groups include aryl lower alkoxycarbonyl groups such as p-methoxybenzyloxycarbonyl, p-nitrobenzyloxycarbonyl and diphenylmethoxycarbonyl; lower alkoxycarbonyl groups such as t-butoxycarbonyl; and lower haloalkoxycarbonyl groups such as 2,2,2-trichloroethoxycarbonyl. The carboxyl blocking group may if desired be removed by any of the appropriate methods disclosed in the literature; thus, for example,

acid or base catalysed hydrolysis is applicable in many cases, as are enzymically-catalysed hydrolyses.

The antibiotic compounds of formula (Ia) and non-toxic salts and non-toxic metabolically labile esters thereof may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds may be formulated for injection and may be presented in unit dose form, in ampoules, or in multi-dose containers, if necessary with an added preservative. The compositions may also take such forms as suspensions, solutions, or emulsions in oily or aqueous vehicles, and may contain formulatory agents such as suspending, stabilising and/or dispersing agents. Alternatively the active ingredient may be in powder form for reconstitution with a suitable vehicle, e.g. sterile, pyrogen-free water, before use.

If desired, such powder formulations may contain an appropriate non-toxic base in order to improve the water-solubility of the active ingredient and/or to ensure that when the powder is reconstituted with water, the pH of the resulting aqueous formulation is physiologically acceptable. Alternatively the base may be present in the water with which the powder is reconstituted. The base may be, for example, an inorganic base such as sodium carbonate, sodium bicarbonate or sodium acetate, or an organic base such as lysine or lysine acetate.

The compositions may also be presented in a form suitable for absorption by the gastro-intestinal

tract, for example tablets, capsules, syrups, or suspensions for oral administration, and suppositories.

Compositions for veterinary medicine may, for example, be formulated as intramammary preparations in either long acting or quick-release bases.

The compositions may contain from 0.1% upwards, e.g. 0.1-99% of the active material, depending on the method of administration. When the compositions comprise dosage units, each unit will preferably contain 100-3000 mg of the active ingredient e.g. 200-2000 mg . The daily dosage for adult human treatment will preferably range from 500 to 12000 mg e.g. 1000-9000 mg per day, depending *inter alia* on the nature of the infection and the route and frequency of administration. In general, intravenous or intramuscular administration will be employed, for example using 400 to 4000 mg per day of the active ingredient in adult human treatment. In treating *Pseudomonas* infections higher daily doses may be required. It will be appreciated that in some circumstances, for example, in the treatment of neonates, smaller dosage units and daily dosages may be desirable.

The antibiotic compounds according to the invention may be administered in combination with other therapeutic agents such as antibiotics, for example penicillins or other cephalosporins.

The following Examples illustrate the invention. All temperatures are in °C. Sorbsil U30 is silica gel manufactured by Joseph Crosfield and Son of Warrington, Lancashire, England. Amberlite XAD-2 is a non-functional macroreticular resin manufactured by Rohm and Haas of Philadelphia, U.S.A.

**Preparation 1**

**Ethyl (Z)-2-Cyclopropylmethoxyimino-2-(2-tritylaminothiazol-4-yl) acetate**

Ethyl (Z)-2-hydroxyimino-2-(2-tritylaminothiazol-4-yl)acetate, hydrochloride salt (30g) was stirred with cyclopropylmethyl bromide (13.5g) in dimethylsulphoxide (150 ml) containing potassium carbonate (30g)

under nitrogen at 21° for 7 hours. The mixture was partitioned between methylene chloride and water. The aqueous layer was extracted with more methylene chloride and the combined organic solutions were washed with water. After drying with magnesium sulphate, the solution was concentrated and loaded onto a column of Sorbsil U30 silica gel (200g). The column was eluted with ethyl acetate (10 to 30%) in petroleum ether (b.p. 40-60°). Evaporation of appropriate fractions gave the title compound (20.9g); $\lambda_{max}$ (ethanol) 234.5nm ($E^{1\%}_{1cm}$ 403); $\lambda_{inf.}$ 254.5nm ($E^{1\%}_{1cm}$ 302), 259.5nm ($E^{1\%}_{1cm}$ 267), 265nm ($E^{1\%}_{1cm}$ 229), 271.5nm ($E^{1\%}_{1cm}$ 190) and 294nm ($E^{1\%}_{1cm}$ 111); $\nu_{max}$ ($CHBr_3$) 3398 (NH), 1730 (ester), and 1593 and 1491cm$^{-1}$ (aromatic double bond).

Preparation 2

(Z)-2-Cyclopropylmethoxyimino-2-(2-tritylaminothiazol-4-yl)acetic acid.

The product of Preparation 1 (20g) was dissolved in ethanol (200 ml) and sodium hydroxide (3.12g) in water (40ml) was added. The mixture was refluxed for 45 minutes during which precipitation occurred. Some of the ethanol (ca 150ml) was distilled off and the residue was cooled. The mixture was partitioned between methylene chloride and water containing 2N hydrochloric acid (70 ml). The organic layer was washed with water, each aqueous layer being back-extracted with more methylene chloride. The combined organic layers were dried with magnesium sulphate and evaporated to give the title compound (20g); $\lambda_{inf.}$ (ethanol) 234.5nm ($E^{1\%}_{1cm}$ 383) 259.5nm ($E^{1\%}_{1cm}$ 242), 266.5nm ($E^{1\%}_{1cm}$ 226) and 272.5nm ($E^{1\%}_{1cm}$ 217); $\nu_{max}$ (Nujol) 3260 (NH) and 1685cm$^{-1}$ (acid).

Example 1

t-Butyl (6R,7R)-3-Acetoxymethyl-7-[(Z)-2-(cyclopropylmethoxyimino)-2-(2-tritylaminothiazol-4-yl)acetamido]-ceph-3-em-4-carboxylate

Oxalyl chloride (0.74 ml) was added to a solution of dimethylformamide (0.76ml) in methylene chloride

(20ml) at -20° with stirring under nitrogen. After 10 minutes stirring with ice-water cooling, the mixture was recooled to -20° and (Z)-2-cyclopropylmethoxyimino-2-(2-tritylaminothiazol-4-yl)acetic acid (3.88 g) was added. After 10 minutes with water cooling the solution was recooled to -20° and a solution of t-butyl 3-acetoxymethyl-7-aminoceph-3-em-4-carboxylate (3.28g) in methylene chloride (20ml) containing N,N-dimethylaniline (2.52 ml) was added. The solution was stirred at 21° for one hour. The solution was then washed successively with dilute hydrochloric acid and water, each time back extracting with methylene chloride. The combined organic solutions were dried with magnesium sulphate, concentrated, and chromatographed on Sorbsil U30 (150g) in ethyl acetate [10 to 40% in petroleum ether (bp 40 - 60°)] to give the <u>title compound</u> (5.79g); $[\alpha]_D^{21}$ +25.3° (<u>c</u>1.11, $CHCl_3$), $\nu_{max}$($CHBr_3$) 3400 (NH), 1790 (β-lactam), 1729 (esters), and 1689 and 1512 $cm^{-1}$ (amide).

<u>Example 2</u>

<u>(6R,7R)-3-Acetoxymethyl-7-[(Z)-2-(2-aminothiazol-4-yl)-2-cyclopropylmethoxyimino]acetamidoceph-3-em-4-carboxylic acid, hydrochloride salt</u>

The product of Example 1 (5.60g) was dissolved in formic acid (22ml) and concentrated hydrochloric acid (1.69ml) was added. The solution was stirred at 21° for one hour and the mixture was filtered. The filter-cake was leached with formic acid and the combined filtrates were added to stirred diisopropyl ether (620ml). The precipitate was collected by filtration, washed with diisopropyl ether and dried to give the <u>title compound</u> (3.37g), $[\alpha]_D^{21}$ +37.25° (<u>c</u> 0.59, DMSO), $\nu_{max}$ (Nujol) 3700-2300 ($NH_3^+$ and OH), 1780 (β-lactam), 1725 (ester and acid) and 1662 and 1543 $cm^{-1}$ (amide).

Example 3

(6R,7R)-7-[(Z)-2-(2-Aminothiazol-4-yl)-2-cyclopropylmethoxyiminoacetamido]-3-hydroxymethyl-ceph-3-em-4-carboxylic Acid, Sodium Salt

A suspension of (6R,7R)-3-acetoxymethyl-7-[(Z)-2-(2-aminothiazol-4-yl)-2-cyclopropylmethoxyiminoacetamido ceph-3-em-4-carboxylic acid (2.230 g) in 0.1*M* pH 6.5 phosphate buffer was treated with 2 *M* - sodium hydroxide solution to raise the pH to 6.8. Dry yellow yeast (Rhodosporidium toruloides, CBS 349) (247 mg) was added and the mixture was stirred at 20° for 2 hours, keeping the pH at 6.8 with sodium hydroxide solution. The yeast was removed by filtratiion and the filtrate was run onto a column of Amberlite XAD-2 resin (1kg) in water. The column was washed with water and the product was then eluted with water containing 25% ethanol, to give a solution which was evaporated to give a solid. This solid was triturated with ether to give the title compound (1.066g) as a solid; $[\alpha]_D^{20}$ +79° (*c* 0.3 in dimethylsulphoxide); $\nu_{max}$ 3560, 3450, 3330, 3260 ($NH_2$, NH, OH), 1756 and 1742 (β-lactam,), 1656 and 1542 (CONH) and 1590 $cm^{-1}$ ($CO_2^-$).

Example 4

(6R,7R)-7-[(Z)-2-Cyclopropylmethoxyimino-2-(2-tritylaminothiazol-4-yl)-acetamido]-3-hydroxymethylceph-3-em-4-carboxylic Acid

Oxalyl chloride (0.50 ml) was added under nitrogen to a solution of N,N-dimethylformamide (0.48 ml) in dichloromethane (13 ml) at - 20°. The mixture was stirred at 0°C for 10 minutes and cooled to -20°. (Z)-2-Cyclopropylmethoxyimino-2-(2-tritylaminothiazol-4-yl) acetic acid (2.528g) was added and the solution was stirred at 0° for 20 minutes. The solution was added to a solution of (6R,7R)-7-amino-3-hydroxymethylceph-3-em-4-carboxylic acid (1.110 g) in industrial methylated spirits (20 ml), water 2 ml) and triethylamine (5 ml) at 0°. This mixture was stirred at 0° for 30 minutes and evaporated. The solid was stirred with

water (20 ml), ethyl acetate (20 ml) and methyl isobutyl ketone (20 ml). The pH of this mixture was adjusted to 3.0 with 20% aqueous phosphoric acid. The solid was washed with water, ethyl acetate,and then ether and was dried to give the title acid containing 0.67 mole triethylamine. (1.736 g); $[\alpha]_D^{20}$ +39° (c 0.75 in dimethylsulphoxide; u.v.(ethanol) had $\lambda_{max}$ 238 nm ($E^{1\%}_{1cm}$ 327) and $\lambda_{infl}$ 259 nm ($E^{1\%}_{1cm}$ 250).

Example 5

Diphenylmethyl (6R,7R)-3-Bromomethyl-7-[(Z)-2-cyclopropylmethoxyimino-2-(2-tritylaminothiazol-4-yl)acetamido]-ceph-3-em-4-carboxylate

Oxalyl chloride (0.37ml) was added to a solution of N,N-dimethylformamide (0.38ml) in methylene chloride (10ml) at -20° with stirring under nitrogen. The mixture was stirred with ice-water cooling for ten minutes before recooling to -20°. (Z)-2-Cyclopropylmethoxyimino-2-(2-tritylaminothiazol-4-yl)acetic acid (1.94g) was added and the solution was stirred with ice-water cooling for ten minutes before recooling to -20°. A suspension of diphenylmethyl (6R,7R)-7-amino-3-bromomethylceph-3-em-4-carboxylate (1.98g) in methylene chloride (10ml) containing N,N-dimethylaniline (1.76ml) was added. A clear solution formed as the mixture was allowed to warm to 21° over one hour. The solution was washed with dilute hydrochloric acid and water, each wash being back extracted with more methylene chloride and the combined extracts were dried with magnesium sulphate and evaporated to a small volume. This solution was filtered through Sorbsil U30 (50g) in ethyl acetate and the eluate was evaporated. The residue (3.54g) was crystallised from diethyl ether - petroleum ether (bp 40 to 60°) to give the title compound (2.43g) mp 135 to 147°, $[\alpha]_D^{21}$ -11.9° (c 0.6, $CHCl_3$)

Example A

Dry Powder for Injection

| | |
|---|---|
| (6R,7R)-7-[(Z)-2-(2-aminothiazol-4-yl)-2-cyclopropylmethoxyiminoacetamido]-3-acetoxymethylceph-3-em-4-carboxylic acid | 1.00g |
| Sodium carbonate (anhydrous) | 0.140g |

Blend the sterile cephalosporin antibiotic aseptically with the sodium carbonate. Fill the blend aseptically into glass vials, such that each vial contains an amount equivalent to 500mg of the cephalosporin antibiotic. Purge the vial headspaces with sterile nitrogen and close the vials using rubber discs or plugs, and metal overseals applied by crimping. Constitute the product shortly before administration by dissolving in Water for Injections or other suitable sterile vehicle.

## CLAIMS

1. Cephalosporin compounds of the general formula

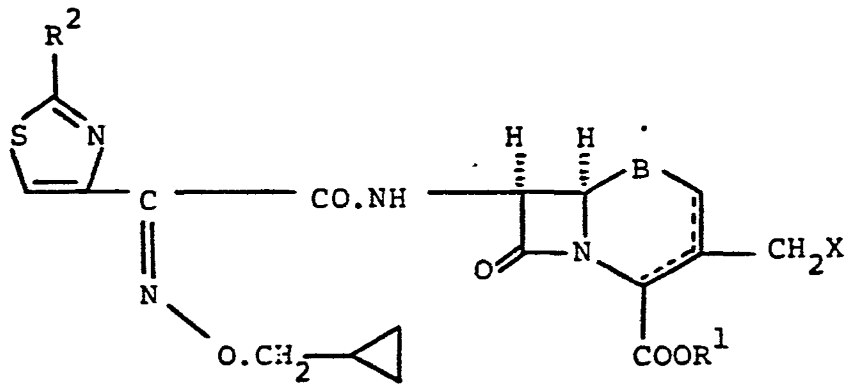

(I)

(wherein $R^1$ represents a hydrogen atom or a carboxyl blocking group, $R^2$ represents an amino or protected amino group, X represents a halogen atom or a hydroxyl or acetoxy group, B is $>S$ or $>S \rightarrow O$, and the dotted line bridging the 2-, 3- and 4-positions indicates that the compound is a ceph-2-em or ceph-3-em compound) and salts thereof.

2. Compounds according to claim 1 of general formula

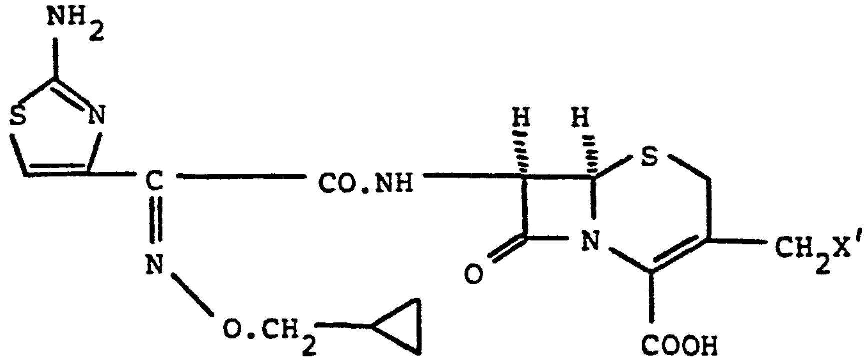

(Ia)

(wherein X' is a hydroxy or acetoxy group) and non-toxic salts and non-toxic metabolically labile esters thereof.

3. A process for the preparation of compounds according to either of claims 1 and 2 which comprises

(A) acylating a compound of the formula

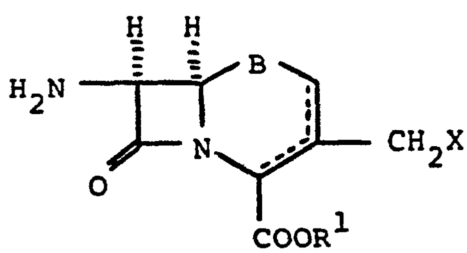

(II)

(wherein $R^1$, X, B and the dotted line are as defined in claim 1) or a salt or N-silyl derivative thereof, with an acid of formula

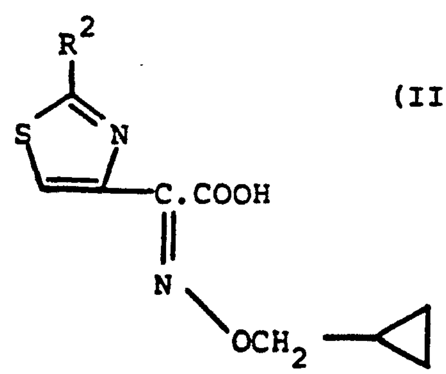

(III)

(wherein $R^2$ is as defined in claim 1) or a salt thereof, or with an acylating agent corresponding thereto;

(B) (for the preparation of compounds in which X is an acetoxy group) reacting a compound of general formula (I) in which X is a hydroxyl group or a salt thereof, with an acetylating agent serving to form an acetoxymethyl group at the 3-position; or

(C) (for the preparation of a compound in which X is a hydroxyl group) 3-deacetylating a compound of formula (I) in which X is an acetoxy group or a salt thereof.

4. A process according to claim 3 for the preparation of a compound according to claim 2 wherein if necessary after process (A), (B) or (C) any of the following reactions in any appropriate sequence, are carried out

i) conversion of a $\Delta^2$-isomer into the corresponding $\Delta^3$-isomer

ii) reduction of a compound wherein B is $>S\rightarrow O$ to form a compound wherein B is $>S$,

iii) conversion of a carboxyl group into an ester function,

iv) formation of a salt function, and

v) removal of any carboxyl-blocking and/or N-protecting groups.

5. A pharmaceutical composition comprising as active ingredient a compound according to claim 2 in association with a pharmaceutical carrier or excipient.

6. Use of a compound according to claim 1 in the preparation of a cephalosporin antibiotic possessing a syn 2-(2-aminothiazol-4-yl)-2-cyclopropylmethoxyiminoacetamido group at the 7β-position.

7. Use according to claim 6 wherein the cephalosporin antibiotic is selected from:-

(6R,7R)-7-[(Z)-2-(2-aminothiazol-4-yl)-2-cyclopropylmethoxyiminoacetamido]-3-(1-pyridiniummethyl)ceph-3-em-4-carboxylate (in which the pyridinium group may be optionally substituted by a 3- or 4-carbamoyl group), (6R,7R)-3-carbamoyloxymethyl-7-[(Z)-2-(2-aminothiazol-4-yl)-2-cyclopropylmethoxyiminoacetamido]ceph-3-em-4-carboxylic acid (in which the carbamoyl group is optionally N-substituted by a methyl or 2-chlorethyl

group), and non-toxic salts and non-toxic metabolically labile esters thereof.